# EUROPÄISCHE PATENTSCHRIFT

(11) **EP 2 929 871 B1**
(45) Veröffentlichungstag und Bekanntmachung des Hinweises auf die Patenterteilung: **11.09.2019**
(21) Anmeldenummer: 15163122.3
(22) Anmeldetag: 10.04.2015
(51) Int. Cl.: A61K 6/00

(54) **HAFTCREMEBASIS FÜR ZAHNPROTHESEN**
CREAM FOR DENTURE ADHESIVE
CRÈME ADHÉSIVE POUR PROTHÈSE DENTAIRE

(30) Priorität: 10.04.2014 CH 5542014
(43) Veröffentlichungstag der Anmeldung: 14.10.2015
(73) Patentinhaber: Bogaert, Jean-Pierre, Monaco (MC)
(72) Erfinder: Marc, Bogaert, 9568 Wildhaus (CH)
(74) Vertreter: Riederer Hasler & Partner Patentanwälte AG

(56) Entgegenhaltungen:
- EP-A2- 2 641 583
- CH-A1- 703 109
- US-A- 5 561 177
- US-A1- 2003 003 195
- Anonymous: "Vegetable Oil", WIKIPEDIA , 5 April 2014 (2014-04-05), pages 1-12, XP009506555, Retrieved from the Internet: URL:https://web.archive.org/web/2014040501 4301/https://en.wikipedia.org/wiki/Vegetab le_oil [retrieved on 2018-06-29]

## Beschreibung

### Gebiet der Erfindung

Die Erfindung betrifft eine Cremebasis (d.h. eine Grundzusammensetzung als Basis für eine Creme), wie im Oberbegriff von Anspruch 1 definiert. Die Zusammensetzung der Cremebasis basiert auf pflanzlichen Ölen und/oder Fetten. Die Erfindung betrifft gemäss einer vorteilhaften Ausführung eine Basis für eine Haftcreme für Zahnprothesen (d.h. eine Haftcremebasis). In einer beispielhaften Ausführung betrifft die Erfindung insbesondere eine Zusammensetzung einer Haftcreme für Zahnprothesen.

### Stand der Technik

Eine Hautcreme besteht üblicherweise aus einer wässrigen (hydrophilen) und einer öligen bzw. fetten (lipophilen) Phase, von der die eine emulsionsartig in der anderen verteilt ist. Der Wasseranteil einer Creme beeinflusst deren Konsistenz. Durch Einbringen feiner Feststoffe kann eine pastöse Konsistenz erzielt werden.

Typische Bestandteile der lipophilen Phase sind auf Mineralöl basierende Paraffine oder synthetische Glyceride. Auch pflanzliche Fette und Öle und tierische Wachse werden eingesetzt. Teilweise werden chemisch modifizierte Öle wegen ihrer besseren Haltbarkeit und Konsistenz verwendet. Um die emulsionsartigen Cremestrukturen zu stabilisieren, werden Emulgatoren zugesetzt. Cremen können weiter Konservierungsmittel, Antioxidantien, Feuchthaltemittel, Konsistenzverbesserer, Spreitverbesserer und Parfümstoffe etc. enthalten (Quelle: Wikipedia zum Begriff Hautcreme).

Kommerzielle Haftcremezusammensetzungen für Zahnprothesen basieren häufig auf einem Gemisch von raffinierten Paraffinen, wasserlöslichen Cellulosederivat-Polymeren und Alkylvinylether/Maleinsäureanhydrid-Copolymeren. Für gewöhnlich werden Mineralöle und mineralische Fette, insbesondere Vaseline verwendet. Mineralöle und mineralische Fette machen üblicherweise ca. 40 Gewichtsprozent (Gew.-%) oder mehr der Haftcremezusammensetzung aus.

Produkte mit cremigen Eigenschaften für kosmetische sowie medizinische Anwendungen sowie Haftcremen für Zahnprothesen werden auch heute noch hauptsächlich auf Mineralöl- und Mineralfettgemischbasis aus gesättigten Kohlenwasserstoffen hergestellt.

Bestandteile dieser Produkte können bei der Anwendung in den Körper gelangen. Insbesondere bei oralen Anwendungen wie z.B. einer Haftcreme für Zahnprothesen ist eine starke Aufnahme in den Körper zu erwarten. Die Basisbestandteile Vaseline und Mineralöl sind nach neuesten Forschungsergebnissen nicht völlig unbedenklich. Vom Anwender werden heute hohe Ansprüche gestellt, insbesondere werden immer mehr Produkte gewünscht, welche möglichst frei von künstlichen und/oder potentiell schädlichen Stoffen sind. Ein Ersatz für Mineralöle sowie mineralische Fette wäre daher wünschenswert.

In den Offenbarungen EP 2 525 768 und EP 2 525 769 werden Haftcremen für Zahnprothesen präsentiert, welche auf pflanzlichen Ölen und Fetten basieren. Insbesondere ist ein hoher Anteil an Ölen und Fetten mit ungesättigten Fettsäuren enthalten. Gemäss US 5,561,177 haben Öle, die ungesättigte Fettsäureester enthalten, den Nachteil, dass sie bei erhöhten Temperaturen oder nach einer gewissen Lagerung instabil werden. Zudem sind Haftcremen, die solche Öle enthalten, aufgrund der vorhandenen ungesättigten Doppelbindungen weniger fest, d.h. zu flüssig für die Anwendung als Haftcreme. Insbesondere die Haftungszeit von Zahnprothesen am Gaumen kann nach EP 2 525 768 durch Zugabe von Trihydroxystearin entscheidend verbessert werden.

Es stellt sich jedoch heraus, dass die Langzeitstabilität noch immer ungenügend ist. Die Langzeitstabilität ist zudem nicht nur bei Haftcremen für Zahnprothesen relevant, sondern allgemein für alle cremeartigen Produkte welche auf pflanzlichen Ölen und Fetten basieren, da viele Cremen über eine längere Zeit (z.B. mehrere Monate bis Jahre) in mehr oder weniger regelmässigen Abständen angewendet werden.

### Aufgabe der Erfindung

Eine Aufgabe der vorliegenden Erfindung liegt darin, eine Grundzusammensetzung für Cremen (d.h. eine Cremebasis) auf pflanzlicher Basis zu formulieren, die eine gute Langzeitstabilität zum Einen der Grundzusammensetzung selbst und zum Anderen der fertig gemischten Creme gewährleistet. Im Weiteren soll die Grundzusammensetzung auf vorwiegend natürlichen und physiologisch unbedenklichen Ausgangsprodukten basieren. Insbesondere soll eine Formulierung bereitgestellt werden, welche mineralölfrei und vorzugsweise auch frei von metallhaltigen Verbindungen wie z.B. Zinkverbindungen ist. Eine besondere Aufgabe ist es, eine in physiologischer Hinsicht verbesserte Grundzusammensetzung für eine Haftcreme, insbesondere eine verbesserte Haftcreme zu erzeugen, die bei guten Haftungseigenschaften (d.h. insbesondere bei konstant hoher Haftungsstärke) eine hohe Langzeitstabilität aufweist und im Vergleich mit den kommerziellen Haftcremen auf Mineralölbasis zumindest gleichwertig ist. In einer Grundzusammensetzung für Cremen, welche dadurch charakterisiert ist, dass mit dem Anteil an pflanzlichen Ölen und/oder Fetten ein gewisser Anteil (i.e. ein gewisser Mindestanteil) an ungesättigten Fettsäuren vorliegt, soll die Langzeitstabilität verbessert werden. Im Speziellen ist es weiter Aufgabe der Erfindung, bei hoher Langzeitstabilität unter Beibehaltung guter Haftungseigenschaften einer Haftcremezusammensetzungen die Langzeitstabilität bei einem Gehalt von mindestens 20 Gewichtsprozent ungesättigter Fettsäuren des pflanzlichen Öls bzw. Fetts zu verbessern.

### Beschreibung

Erfindungsgemäss wird die Aufgabe, wie in Anspruch 1 definiert, mit einer Cremebasis (auch Grundzusammensetzung genannt) dadurch gelöst, dass die Cremebasis die folgenden Komponenten beinhaltet: wenigstens ein erstes pflanzliches Öl oder Fett (d.h. ein Öl oder Fett pflanzlicher Art, d.h. pflanzlichen Ursprungs), dessen Fettsäuregehalt zumindest zu 20 Gew.-% aus ungesättigten (d.h. mehrfach- und einfachungesättigten) Fettsäuren besteht, und ein zweites pflanzliches Öl oder Fett, welches hydriert, vorzugsweise vollhydriert ist. Meist bevorzugt wird insbesondere, dass das hydrierte pflanzliche Öl oder Fett ein vollhydriertes pflanzliches Öl oder Fett ist. Das wenigstens eine erste pflanzliche Öl oder Fett, dessen Fettsäuregehalt zumindest zu 20 Gew.-% aus ungesättigten Fettsäuren besteht, ist insbesondere nicht hydriert.

Pflanzenöle (pflanzliche Öle) sind aus Ölpflanzen gewonnene Fette und fette Öle. Die Abgrenzung zwischen fetten Ölen und Fetten ist die Fliessfähigkeit bei Zimmertemperatur. Je nachdem, ob ein Fett bei Raumtemperatur fest oder flüssig ist, spricht man von Fett oder fettem Öl.

Der Begriff Fettsäure wird hier und im Folgenden in der Bedeutung eines Fettsäurerests gebraucht. Ein pflanzliches Öl oder Fett ist ein Tri-Ester höherer Fettsäuren mit Glycerol, also ein Fettsäureglyzerolester mit drei langkettigen Fettsäuren, welche auch Fettsäurereste genannt werden. Mit Fettsäuregehalt sind im Nachfolgenden (wenn nichts anderes angezeigt ist) insbesondere die als Ester gebundenen Fettsäuren zu verstehen. Höhere oder langkettige Fettsäuren sind hierin definiert als Fettsäuren mit zwölf oder mehr C-Atomen.

Die erfindungsgemässe Zusammensetzung einer Cremebasis kann als Basis für eine Creme, z.B. für die äussere Anwendung, z.B. auf Haut oder Lippen, oder als Basis für eine Haftcreme für Zahnprothesen verwendet werden. Besonders interessant sind Anwendungen, bei welchen eine gute Haftung der Creme auch unter feuchten oder nassen Bedingungen gefordert wird, d.h. z.B. für wasserresistente Cremen, wie z.B. Lippenstift oder Sonnencreme.

Das zweite pflanzliche Öl oder Fett ist vorzugsweise vollhydriert. Das hydrierte pflanzliche Öl kann prinzipiell teilhydriert sein. Zumindest aufgrund gesundheitlicher Betrachtungen ist eine Vollhydrierung jedoch vorzuziehen.

Das Mass der Hydrierung des genannten hydrierten pflanzlichen Öls oder Fetts ist vorzugsweise derart, dass dessen Steigschmelztemperatur (auch Steigschmelzpunkt genannt) zumindest dem Mittelwert (in °C) zwischen der Steigschmelztemperatur des Öls oder Fetts in seinem noch unbehandelten, d.h. nichthydrierten, Zustand und der Steigschmelztemperatur in seinem vollhydrierten Zustand entspricht oder höher ist. Das Mass der Hydrierung ist weiter bevorzugt derart, dass die Steigschmelztemperatur eines (teil-) hydrierten Öls oder Fetts im oberen Viertel des Temperaturbereichs (in °C) liegt, welcher sich zwischen der Steigschmelztemperatur des Öls oder Fetts in seinem noch unbehandelten, d.h. nichthydrierten, Zustand und der Steigschmelztemperatur des Öls oder Fetts in seinem vollhydrierten Zustand ergibt. Das hydrierte pflanzliche Öl oder Fett weist vorzugsweise keine oder kaum Transfettsäuren auf. Dies ist insbesondere der Fall wenn das hydrierte pflanzliche Öl oder Fett vollhydriert ist.

Besonders gute Langzeitstabilitätswerte der Cremebasis können erreicht werden, wenn das Gewichtsverhältnis vom zweiten pflanzlichen Öl oder Fett (d.h. dem hydrierten pflanzlichen Öl oder Fett) zum ersten pflanzlichen Öl oder Fett (d.h. dem pflanzlichen Öl oder Fett, dessen Fettsäuregehalt zumindest zu 20 Gew.-% aus ungesättigten Fettsäuren besteht) 1:450 oder mehr, bevorzugt 1:190 oder mehr, weiter bevorzugt 1:150 oder mehr, weiter bevorzugt 1:100 oder mehr, weiter bevorzugt 1:50 oder mehr, beträgt.

Besonders gute Langzeitstabilitätswerte bei gleichzeitig besonders hoher Haftungsdauer der Cremebasis bzw. einer daraus gebildeten Haftcreme für Zahnprothesen können erreicht werden, wenn das Gewichtsverhältnis vom zweiten pflanzlichen Öl oder Fett (d.h. dem hydrierten pflanzlichen Öl oder Fett) zum ersten pflanzlichen Öl oder Fett (d.h. dem pflanzlichen Öl oder Fett, dessen Fettsäuregehalt zumindest zu 20 Gew.-% aus ungesättigten Fettsäuren besteht) im Bereich von 1:450 bis 1:10, bevorzugt von 1:190 bis 1:13, weiter bevorzugt von 1:150 bis 1:14, weiter bevorzugt von 1:100 bis 1:15, weiter bevorzugt von 1:50 bis 1:16, liegt.

Hier und im Weiteren dort wo bevorzugte Bereiche angegeben sind, ergeben sich weitere bevorzugte Bereiche aus Kombinationen der in den Bereichen genannten Minima und Maxima.

Pflanzliche Öle und Fette werden aus der Saat bzw. Frucht von Ölpflanzen gewonnen. Chemisch sind Pflanzenöle und Fette Ester des Glycerins mit Fettsäuren, häufig mit drei Fettsäuren, sogenannte Triglyceride. Viele pflanzliche Öle und Fette werden vom Menschen regelmässig mit der Nahrung aufgenommen und sind in den üblicherweise in der Nahrung vorkommenden Mengen physiologisch völlig unbedenklich. Beispiele für die in der erfindungsgemässen Cremebasiszusammensetzung verwendbaren Öle und Fette sind Olivenöl, Rapsöl, Erdnussöl, Maisöl, Weizenkeimöl, Walnussöl, Traubenkernöl, Sonnenblumenöl, Weizenkeimöl, Sesamöl, Palmöl, Palmkernöl, Mohnöl, Leinöl, Kürbiskernöl, Distelöl, Nachtkerzenöl, Hanföl und Kokosnussfett. Hierbei wird Olivenöl bevorzugt verwendet, da dieses zu einer besonders physiologisch verträglichen und wenn für Haftcremen eingesetzt geschmacklich akzeptierten Produkten führt. Bekanntermassen ist raffiniertes Olivenöl geschmacklich relativ neutral, leicht erhältlich und allgemein als gesund und bekömmlich bekannt. Olivenöl wirkt antiseptisch und antibakteriell. Olivenöl kann das Wachstum von Keimen (Bakterien, Pilze) zwischen Gaumen und Prothese deutlich verlangsamen. Der Pilz Candida Albicans kann mit Hilfe einer mit Olivenöl angereicherten Haftcreme bekämpft werden (siehe auch EP 2 525 768 oder EP 2 525 769).

Folglich wird erfindungsgemäss bevorzugt, dass das pflanzliche Öl oder Fett, dessen Fettsäuregehalt zumindest zu 20 Gew.-% aus ungesättigten Fettsäuren besteht, mehrheitlich ein Olivenöl beinhaltet.

Das hydrierte Öl oder Fett beinhaltet im Wesentlichen hydriertes Sojaöl oder hydriertes Rizinusöl, insbesondere jeweils vollhydriert. Besonders bevorzugt besteht das hydrierte Öl oder Fett aus hydriertem Sojaöl, insbesondere vollhydriertem Sojaöl.

Das hydrierte Sojaöl hat (vor Zugabe) vorzugsweise eine Steigschmelztemperatur von mindestens 30°C, weiter bevorzugt mindestens 50 °C. Besonders bevorzugt ist ein im Wesentlichen vollhydriertes Sojaöl. Dieses hat eine Steigschmelztemperatur von mindestens 60°C, vorzugsweise im Bereich von ca. 60 bis 80°C, insbesondere von mindestens 65°C, vorzugsweise im Bereich von 65-75°C, weiter bevorzugt im Bereich von 66-72°C.

Der Fettsäuregehalt des hydrierten pflanzlichen Öls oder Fetts in nahezu vollhydriertem Zustand beinhaltet vorteilhafterweise maximal bis zu 1 Gew.-% mehrfachungesättigte Fettsäuren.

Der Fettsäuregehalt des hydrierten pflanzlichen Öls oder Fetts in vollyhdriertem oder im Wesentlichen vollhydriertem Zustand besteht vorteilhafterweise zu mindestens 50 Gew.-%, bevorzugt mindestens 60 Gew.-%, weiter bevorzugt mindestens 65 Gew.-%, weiter bevorzugt mindestens 70 Gew.-%, aus einfachungesättigten Fettsäuren.

Die Begriffe nahezu oder im Wesentlichen vollhydriert bedeuten insbesondere zumindest zu 95 %, vorzugsweise zu 98 % hydriert; und zeigen dadurch insgesamt einen hohen Hydrierungsanteil an.

Hydriertes Sojaöl hat insbesondere die Eigenschaft bei einem hohen Gehalt an einfach gesättigten Fettsäuren kaum mehrfachungesättigte Fettsäuren zu enthalten.

Die erfindungsgemässe Zusammensetzung der Cremebasis zeichnet sich bevorzugt dadurch aus, dass mindestens 50 Gew.-%, bevorzugt mindestens 60 Gew.-%, weiter bevorzugt mindestens 70 Gew.-% des Fettsäuregehalts des hydrierten Öls oder Fetts Stearinsäure ist.

Zweckmässigerweise besteht der Fettsäuregehalt des Öls oder Fetts, dessen Fettsäuregehalt zumindest zu 20 Gew.-% aus ungesättigten Fettsäuren besteht, aus Fettsäuren mit einer Kettenlänge von 12-26 C-Atomen, bevorzugt 14-24 C-Atomen, weiter bevorzugt von 16-18 C-Atomen.

Weiter bevorzugt besteht der Fettsäuregehalt des pflanzlichen Öls oder Fetts, dessen Fettsäuregehalt zumindest zu 20 Gew.-% aus ungesättigten Fettsäuren besteht, mehrheitlich, vorzugsweise zu wenigstens 65 Gew.-%, weiter bevorzugt zu wenigstens 80 Gew.-%, aus Fettsäuren mit einer Kettenlänge von 12 oder mehr C-Atomen, insbesondere aus Fettsäuren mit einer Kettenlänge von 16-18 C-Atomen.

Es wird bevorzugt, dass der Fettsäuregehalt des pflanzlichen Öls oder Fetts, dessen Fettsäuregehalt wie oben beschrieben zumindest zu 20 Gew.-% aus ungesättigten Fettsäuren besteht, insbesondere zumindest zu 40 Gew.-%, weiter bevorzugt zumindest zu 50 Gew.-%, weiter bevorzugt zumindest zu 60 Gew.-%, weiter bevorzugt zumindest zu 70 Gew.-%, weiter bevorzugt zumindest zu 80 Gew.-% aus ungesättigten (d.h. mehrfach- und einfachungesättigte) Fettsäuren besteht. Hierzu gehört insbesondere das bevorzugte Olivenöl.

Daraus folgt, dass der Fettsäuregehaltsanteil an ungesättigten Fettsäuren entsprechend tief ist. Insbesondere bevorzugt ist, dass der Fettsäuregehalt des pflanzlichen Öls oder Fetts, dessen Fettsäuregehalt zumindest zu 20 Gew.-% aus ungesättigten Fettsäuren besteht, vorzugsweise zu weniger als 30 Gew.-%, weiter bevorzugt zu weniger als 25 Gew.-%, weiter bevorzugt zu weniger als 20 Gew.-%, weiter bevorzugt zu weniger als 15 Gew.-%, aus gesättigten Fettsäuren besteht.

Vorzugsweise besteht der Fettsäuregehalt des pflanzlichen Öls oder Fetts, dessen Fettsäuregehalt zumindest zu 20 Gew.-% aus ungesättigten Fettsäuren besteht, insgesamt zumindest zu 8 Gew.-%, bevorzugt zumindest zu 9 Gew.-%, weiter bevorzugt zumindest zu 10 Gew.-%, aus mehrfachungesättigten Fettsäuren. Bevorzugt besteht der Fettsäuregehalt des pflanzlichen Öls oder Fetts, dessen Fettsäuregehalt zumindest zu 20 Gew.-% aus ungesättigten Fettsäuren besteht, insgesamt maximal zu 30 Gew.-%, bevorzugt maximal zu 25 Gew.-%, weiter bevorzugt maximal zu 20 Gew.-%, aus mehrfachungesättigten Fettsäuren. Weiter bevorzugte Bereiche werden durch je einen der genannten Mindest- und Maximalwerte gebildet, wobei der engste Bereich der bevorzugteste ist.

Das erste pflanzliche Öl oder Fett ist in der Cremebasis vorzugsweise zu 70-97 Gew.-%, weiter bevorzugt zu 80-96 Gew.-%, weiter bevorzugt zu 85-95 Gew.-%, bezogen auf die Gesamtmenge der Cremebasis enthalten.

Das zweite pflanzliche Öl oder Fett, insbesondere ein hydriertes Sojaöl, ist in der Cremebasis vorzugsweise bis maximal 20 Gew.-%, bevorzugt im Bereich von 0.1 bis 10 Gew.-%, weiter bevorzugt 0.2 bis 8 Gew.-%, weiter bevorzugt 0.5 bis 6.5 Gew.-%, weiter bevorzugt 1 bis 6 Gew.-%, bezogen auf die Gesamtmenge der Cremebasis enthalten.

Weitere Substanz ausgewählt aus der Gruppe bestehend aus Trihydroxistearin, Phosphoglyceriden und Siliziumdioxid können in der erfindungsgemässen Zusammensetzung der Cremebasis enthalten sein.

Vorzugsweise sind die Phosphoglyceride zumindest aus der Gruppe der Lecithine ausgewählt, wobei insbesondere Soyalecithin günstig ist.

Das Trihydroxystearin liegt zweckmässigerweise in einer Menge von 0.001 Gew.-% bis 5 Gew.-% und bevorzugt in einer Menge von 0.01 Gew.-% bis 3 Gew.-%, bezogen auf die Gesamtmenge der Zusammensetzung der Cremebasis vor. Weiter zweckmässigerweise liegt das Trihydroxystearin bevorzugt in einer Menge von 0.1 Gew.-% bis 5 Gew.-%, weiter bevorzugt in einer Menge von 0.5 Gew.-% bis 4 Gew.-% und weiter bevorzugt in einer Menge von 1 Gew.-% bis 3 Gew.-%, bezogen auf die Gesamtmenge der Zusammensetzung der Cremebasis vor.

Dass Phosphoglyceride liegt zweckmässigerweise in einer Menge von 0.001 Gew.-% bis 3 Gew.-%, bevorzugt in einer Menge von 0.001 Gew.-% bis 2 Gew.-% und weiter bevorzugt in einer Menge von 0.01 Gew.-% bis 1 Gew.-%, bezogen auf die Gesamtmenge der Zusammensetzung der Cremebasis vor.

Vorteilig ist, wenn Phosphoglyceride und Trihydroxystearin, insbesondere Lecithin und Trihydroxystearin, in Kombination in der erfindungsgemässen Zusammensetzung der Cremebasis vorliegen. Besonders vorteilig ist, wenn Lecithin und Trihydroxystearin in der erfindungsgemässen Zusammensetzung der Cremebasis vorliegen.

Zweckmässigerweise liegen Lecithin in einer Menge von 0.001 Gew.-% bis 3 Gew.-%, vorzugsweise in einer Menge von 0.001 Gew.-% bis 2 Gew.-%, besonders bevorzugt in einer Menge von 0.01 Gew.-% bis 1 Gew.-%, und Trihydroxystearin in einer Menge von 0.001 Gew.-% bis 5 Gew.-%, vorzugsweise in einer Menge von 0.001 Gew.-% bis 4 Gew.-%, weiter bevorzugt 0.01 Gew.-% bis 3 Gew.-%, bezogen auf die Gesamtmenge der Zusammensetzung der Cremebasis vor.

Zweckmässigerweise liegt das Siliziumdioxid in einer Menge von 0.001 Gew.-% bis 5 Gew.-%, bevorzugt in einer Menge von 0.1 Gew.-% bis 4 Gew.-% und weiter bevorzugt in einer Menge von 0.5 Gew.-% bis 3 Gew.-%, bezogen auf die Gesamtmenge der Zusammensetzung der Cremebasis vor. Weiter zweckmässigerweise liegt das Siliziumdioxid bevorzugt in einer Menge von 0.1 Gew.-% bis 5 Gew.-%, weiter bevorzugt in einer Menge von 0.5 Gew.-% bis 4 Gew.-% und weiter bevorzugt in einer Menge von 1 Gew.-% bis 3 Gew.-%, bezogen auf die Gesamtmenge der Zusammensetzung der Cremebasis vor.

Die erfindungsgemässe Zusammensetzung der Cremebasis kann Polyethylenglycole enthalten. Diese liegen gegebenenfalls in einer Menge von 0.001-15 Gew.-%, bevorzugt von 3-12 Gew.-% und weiter bevorzugt von 5-9 Gew.-% bezogen auf die Gesamtmenge der Zusammensetzung der Cremebasis vor. Zudem weisen die Polyethylenglycole vorzugsweise eine Molmasse im Bereich von 100000-7000000 g/mol, insbesondere von 200000-400000 g/mol auf.

Es wird bevorzugt, dass die Menge der enthaltenen Trihydroxystearin, Phosphoglyceride, Siliziumdioxid und gegebenenfalls Polyethylenglycole in Summe die Menge von maximal 10 Gew.-%, bevorzugt maximal 7 Gew.-%, weiter bevorzugt maximal 5 Gew.-% bezogen auf die Gesamtmenge der Zusammensetzung der Cremebasis nicht übersteigt.

Gegebenenfalls kann weiter z.B. Talkum in der erfindungsgemässen Zusammensetzung der Cremebasis enthalten sein.

Die erfindungsgemässe Cremebasis wird insbesondere für kosmetische, medizinische, und/oder dentale Zwecke eingesetzt.

Da die Cremebasis physiologisch verträglich ist kann diese gleichermassen für äussere und innere Anwendungen, d.h. z.B. für äussere topische (auf der Haut) und orale (im Mund) Anwendungen verwendet werden. Die erfindungsgemässe Zusammensetzung der Cremebasis eignet sich somit insbesondere zur Herstellung von kosmetischen Produkten, wie z.B. Cremen, Lippenstiften, Mascara etc. Für medizinische Produkte kann insbesondere ein medizinischer Wirkstoff mit der erfindungsgemässen Cremebasis vermengt werden. Vorteilhafterweise, lassen sich mit der erfindungsgemässen Cremebasis Cremen für die orale Anwendung herstellen.

Im Allgemeinen werden für die erfindungsgemässe Cremebasis lediglich Fettsäuren pflanzlichen Ursprungs verwendet, dadurch ist diese physiologisch besonders verträglich.

Die erfindungsgemässe Haftcreme hat den Vorteil, dass sie aus physiologisch völlig unbedenklichen Komponenten zusammengesetzt ist und daher auch bei längerer Anwendung unbedenklich ist. Überraschenderweise konnte eine Cremebasiszusammensetzung, insbesondere auch eine Cremezusammensetzung, gefunden werden, die trotz eines hohen Anteils an ungesättigten Fettsäuren eine sehr gute und lang andauernde Haftung gewährleistet. Mit den neuen Zusammensetzungen konnte besonders gute Langzeitstabilität (d.h. Lagerstabilität) erreicht werden - zum einen bei einer Cremebasis, zum anderen bei der damit hergestellten Creme, insbesondere der Haftcreme für Zahnersatz.

Die erfindungsgemässe Cremebasis wird bei der Herstellung von Cremen, anteilig vorzugsweise mit mindestens 10 Gewichtsprozent der Menge einer herzustellenden Creme verwendet. Aufgrund der physiologisch kompatiblen Zusammensetzung, kann die erfindungsgemässe Cremebasis anteilig jedoch mit bis zu 100 Gew.-% verwendet werden.

### Besondere Anwendung:

In einer bevorzugten Verwendung der erfindungsgemässen Cremebasis (d.h. der Grundzusammensetzung) wir diese zur Herstellung von einer Haftcreme für Zahnprothesen verwendet. Hierbei werden insbesondere wenigstens ein wasserlösliches Polymer ausgewählt aus der Gruppe der Cellulosederivate, und wenigstens ein Alkylvinylether/Maleinsäureanhydrid-Copolymer mit oben genannter Cremebasis bzw. Haftcremebasis vermischt, um eine Haftcremezusammensetzung zu bilden. Vorteilhafterweise kann die Cremebasis an einem ersten Ort hergestellt werden, während die Mischung der Cremebasis mit den haftcremespezifischen Komponenten, d.h. dem wasserlöslichen Polymer und dem Alkylvinylether/Maleinsäureanhydrid-Copolymer, zu einem späteren Zeitpunkt und gegebenenfalls an einem anderen, zweiten Ort hergestellt werden kann.

Die Cremebasis wird bei der Herstellung von Cremen, welche im Mundraum aufgetragen werden, anteilig vorzugsweise zu zumindest etwa 20 Gewichtsprozent der Menge der herzustellenden Creme verwendet. Bei der Herstellung von Haftcremen für Zahnprothesen macht die Cremebasis vorzugsweise 20 bis 50 Gewichtsprozent der Gesamtmenge der herzustellenden Haftcreme aus.

Eine bevorzugte Haftcremezusammensetzung für Zahnprothesen zeichnet sich dadurch aus, dass diese bezogen auf die Gesamtmenge der Haftcremezusammensetzung wenigstens zu 25 Gew.-% die Cremebasis, zu 10 bis 45 Gew.-% das wenigstens eine wasserlöslichen Polymer ausgewählt aus der Gruppe der Cellulosederivate, und zu 25 bis 45 Gew.-% das wenigstens eine Alkylvinylether/Maleinsäureanhydrid-Copolymer enthält.

Eine besonders bevorzugte Haftcremezusammensetzung für Zahnprothesen zeichnet sich dadurch aus, dass diese bezogen auf die Gesamtmenge der Haftcremezusammensetzung wenigstens zu 25 Gew.-% die Cremebasis, zu 25 bis 45 Gew.-% das wenigstens eine wasserlöslichen Polymers ausgewählt aus der Gruppe der Cellulosederivate, und zu 25 bis 45 Gew.-% das wenigstens eine Alkylvinylether/Maleinsäureanhydrid-Copolymers enthält.

Im Detail beinhaltet eine erfindungsgemässe Haftcreme für Zahnprothesen
a) wenigstens ein pflanzliches Öl oder Fett, dessen Fettsäuregehalt zumindest zu 20 Gew.-% aus mehrfachungesättigten Fettsäuren besteht,
b) wenigstens ein wasserlösliches Polymer ausgewählt aus der Gruppe der Cellulosederivate,
c) wenigstens ein Alkylvinylether/Maleinsäureanhydrid-Copolymer, und
d) wenigstens ein hydriertes pflanzliches Öl oder Fett (welches bevorzugt ein vollhydriertes pflanzliches Öl und weiter bevorzugt hydriertes Sojaöl ist).

Die Haftcreme für Zahnprothesen enthält bevorzugt bezogen auf ihre Gesamtmenge wenigstens 25 Gew.-% des pflanzliche Öls oder Fetts, dessen Fettsäuregehalt zumindest zu 20 Gew.-% aus ungesättigten Fettsäuren besteht.

Die Haftcreme für Zahnprothesen enthält zweckmässigerweise bezogen auf ihre Gesamtmenge zwischen 0.17 Gew.-% und 2.255 Gew.-%, vorzugsweise von 0.2 Gew.-% bis 2.25 Gew.-%, des hydrierten pflanzlichen Öls oder Fetts.

Eine erfindungsgemässe Haftcreme für Zahnprothesen beinhaltet beispielsweise, bezogen auf die Gesamtmenge der Haftcremezusammensetzung,
a) 25-60 Gew.% des pflanzlichen Öls oder Fetts, dessen Fettsäuregehalt zu 20 Gew.-% aus ungesättigten Fettsäuren besteht,
b) 10-40 Gew.-% des wasserlöslichen Polymers ausgewählt aus der Gruppe der Cellulosederivate,
c) 25-45 Gew.-% des wenigstens einen Alkylvinylether/Maleinsäureanhydrid-Copolymers,
d) zwischen 0.17 und 2.255 Gew.-% hydriertes pflanzliches Öl oder Fett (wie oben beschrieben), und gegebenenfalls
e) 0-3 Gew.-% oder bis zu 3 Gew.-% Phosphoglyceride,
f) 0-2.5 Gew.-% oder bis zu 2.5 Gew.-% Trihydroxystearin, und
g) 0-10 Gew.-% weitere Zusatzstoffe.

Die weiteren Zusatzstoffe werden zweckmässigerweise ausgewählt aus der Gruppe der Antioxidantien, Geschmackstoffe, Farbstoffe, Stabilisatoren, Verdicker, Emulgatoren, medizinischen Wirkstoffe, und aus Gemischen davon. Ein Stoff kann hierbei mehreren Wirkungsgruppen angehören, bzw. mehrere Wirkungen zeigen. Diese weiteren Zusatzstoffe liegen in Summe in einer Menge von maximal 10 Gew.-%, bevorzugt maximal 2 Gew.-%, und besonders bevorzugt maximal 1 Gew.-%, bezogen auf die Gesamtmenge der Haftcremezusammensetzung vor. Mit Hinblick auf die physiologische Unbedenklichkeit wird die Zugabemenge von Zusätzen so gering wie möglich gehalten.

Haftcremen mit sehr guten Trageigenschaften und Langzeitstabilitätswerten werden vor allem dann erzielt, wenn das oben erwähnte pflanzliche Öl oder Fett, dessen Fettsäuregehalt zumindest zu 20 Gew.-% aus ungesättigten (d.h. mehrfach- und einfachungesättigten) Fettsäuren besteht, im Wesentlichen Olivenöl ist und das oben erwähnte hydrierte pflanzliche Öl oder Fett im Wesentlichen Sojaöl ist.

Die erfindungsgemässe Haftcreme zeichnet sich somit durch eine gesundheitlich unbedenkliche Zusammensetzung aus, die auf pflanzlichen Ölen und/oder Fetten, insbesondere Olivenöl, basiert. Olivenöl kann hierbei gleichermassen in teilraffiniertem wie auch unraffiniertem Zustand verwendet werden. Vorteilhafterweise wird kaltgepresstes und ohne übermässige Temperatureinwirkung schonend hergestelltes Olivenöl aus erster Pressung verwendet (d.h. natives Olivenöl Extra).

Durch die Verwendung von lediglich Fettsäuren pflanzlichen Ursprungs, ergibt sich der Vorteil, dass die Haftcreme für den Prothesenträger beim Verschlucken besonders verträglich ist.

Die Cellulosederivate sind wasserlösliche Polymere vorzugsweise ausgewählt aus der Gruppe bestehend aus Methylcellulose, Carboxymethylcellulose, Sodiumcarboxymethylcellulose, Hydroxypropylmethylcellulose und Gemischen davon. Bevorzugt wird Carboxymethylcellulose, insbesondere Natriumcarboxymethylcellulose, verwendet. Das wasserlösliche Polymer ausgewählt aus der Gruppe der Cellulosederivate liegt in einer Menge von 15 bis 45 Gew.-% bezogen auf die Gesamtmenge der Zusammensetzung vor. Vorzugsweise liegt das wasserlösliche Polymer ausgewählt aus der Gruppe der Cellulosederivate in einer Menge von 20 bis 40 Gew.-% und weiter bevorzugt in einer Menge von 25 bis 38 Gew.-% bezogen auf die Gesamtmenge der Zusammensetzung vor.

Vorteilhafterweise liegt das Alkylvinylether/Maleinsäureanhydrid-Copolymer zum Teil als Säure, Ester und/oder Salz vor. Üblicherweise sind die Kationen der Salze ausgewählt aus der Gruppe bestehend aus Calcium-, Kalium-, Natrium-, Magnesium-, Aluminium-, Zinksalzen und Gemischen davon, insbesondere aus der Gruppe bestehend aus Ca²⁺, K⁺, Na⁺, Mg²⁺, Al³⁺ und/oder Zn²⁺. Als das Alkylvinylether/Maleinsäureanhydrid-Copolymer wird insbesondere ein Methylvinylether/Maleinsäureanhydrid-Copolymer verwendet. Das Methylvinylether/Maleinsäureanhydrid-Copolymer liegt, zum Beispiel als Salz, Ester und/oder Säure, in einer Menge von 20-45 Gew.-% bezogen auf die Gesamtmenge der Zusammensetzung vor. Vorteilhafterweise liegt das Methylvinylether/Maleinsäureanhydrid-Copolymer, zum Beispiel als Salz und/oder Säure, in einer Menge von 25-45 Gew.-% und bevorzugt 25-40 Gew.-% und weiter bevorzugt 28-40 Gew.-% bezogen auf die Gesamtmenge der Zusammensetzung vor.

Zinkverbindungen liegen vorteilhafterweise nicht vor; d.h. ein Zusatz von Zinkverbindungen ist mit Vorteil ausgeschlossen. Insbesondere um gesundheitliche Risiken aufgrund erhöhter Zinkaufnahme durch Haftcremen zu minimieren, sollte der Zinkgehalt auf eine Obergrenze von 1 Gew.-%, bevorzugt 0.5 Gew.-%, weiter bevorzugt 0.1 Gew.-%, weiter bevorzugt 0.01 Gew.-%, und weiter bevorzugt 0.001 Gew.-%, jeweils bezogen auf die Gesamtmenge der Haftcremezusammensetzung limitiert sein. Bevorzugt wird jedoch die Abwesenheit jeglichen Zinks oder jeglicher Zinkverbindungen.

Während vorstehend spezifische Ausführungsformen beschrieben wurden, ist es offensichtlich, dass unterschiedliche Kombinationen der aufgezeigten Ausführungsmöglichkeiten angewendet werden können, insoweit sich die Ausführungsmöglichkeiten nicht gegenseitig ausschliessen.

Nachfolgend wird die Erfindung anhand von Beispielen verdeutlicht. Die Beispiele präsentieren Zusammensetzungen, die gute Haftkraft und Haftdauer gewährleisten, insbesondere auch bei Unterdruckbedingungen. Die Prozentangaben sind auf zwei Stellen nach dem Komma gerundet.

### Beispiel 1:

Erfindungsgemässes Beispiel einer Cremebasis, welche weiter für die Herstellung einer Haftcreme für Zahnprothesen verwendet wird:

| **Inhaltsstoffe der Cremebasis** | **CAS-Nr.** | **Gewichtsprozent-anteile* bezogen auf die Cremebasis** | **Gewichtsprozent-anteile bezogen auf die Haftcreme** |
|---|---|---|---|
| Olivenöl | 8001-25-0 | 90.94 | 31.0600528 |
| Hydriertes Sojaöl | 8013-70-4 | 4.50 | 1.53693 |
| Trihydroxystearin | 139-44-6 | 2.28 | 0.7802593 |
| Siliziumdioxid | 112945-52-5, 68611-44-9 | 1.99 | 0.6783923 |
| Menthol | 226-51-5 | 0.18 | 0.0601603 |
| Lezithin | 8002-43-5 | 0.05 | 0.0169598 |
| Citrus Limonum | 8008-56-8 | 0.04 | 0.0143912 |
| Menthyl-Lactat | 59259-38-0 | 0.02 | 0.006854 |
| **Total Inhaltsstoffe der Cremebasis** | | **100** | **34.154**** |

| | | | |
|---|---|---|---|
| * Gerundet auf zwei Stellen nach dem Komma. ** Gerundet auf drei Stellen nach dem Komma. | | | |

| **Inhaltsstoffe der Haftcreme** | **CAS-Nr.** | **Gewichtsprozentanteile bezogen auf die Haftcreme** |
|---|---|---|
| Cellulose Gum (insb. CMC 1500) | 9004-32-4 | 35.271 |
| Cremebasis | | 34.154 |
| Calcium/Sodium PVM/MA Copolymer*** | 62 386-95-2 | 30.575 |

| | | |
|---|---|---|
| *** z.B. Gantrez | | |

Die Stabilitätstests ergaben wenig bis keine Phasenseparation nach 2 Monaten. Der Haftungstest zeigte gute Haftungseigenschaften.

### Beispiel 2:

Vergleichsbeispiel einer Cremebasis, welche weiter für die Herstellung einer Haftcreme für Zahnprothesen verwendet wird:

| **Inhaltsstoffe der Cremebasis** | **CAS-Nr.** | **Gewichtsprozent-anteile* bezogen auf die Cremebasis** | **Gewichtsprozent-anteile bezogen auf die Haftcreme** |
|---|---|---|---|
| Olivenöl | 8001-25-0 | 95.44 | 32.5968348 |
| Hydriertes Sojaöl | 8013-70-4 | - | - |
| Trihydroxystearin | 139-44-6 | 2.28 | 0.7802554 |
| Siliziumdioxid | 112945-52-5, 68611-44-9 | 1.99 | 0.678389 |
| Menthol | 226-51-5 | 0.18 | 0.060160 |
| Lezithin | 8002-43-5 | 0.05 | 0.0169597 |
| Citrus Limonum | 8008-56-8 | 0.04 | 0.0143911 |
| Menthyl-Lactat | 59259-38-0 | 0.02 | 0.006854 |
| **Total Inhaltsstoffe der Cremebasis** | | 100 | **34.154**** |

| | | | |
|---|---|---|---|
| * Gerundet auf zwei Stellen nach dem Komma. ** Gerundet auf drei Stellen nach dem Komma. | | | |

| **Inhaltsstoffe der Haftcreme** | **CAS-Nr.** | **Gewichtsprozentanteile bezogen auf die Haftcreme** |
|---|---|---|
| Cellulose Gum (insb. CMC 1500) | 9004-32-4 | 35.271 |
| Creme basis | | 34.154 |
| Calcium/Sodium PVM/MA Copolymer*** | 62 386-95-2 | 30.575 |

| | | |
|---|---|---|
| *** z.B. Gantrez | | |

In den Stabilitätstests zeigte sich nach einem Monat bis zwei Monaten deutliche Phasenseparation. Der Haftungstest zeigte dennoch gute Haftungseigenschaften, wobei die Haftungsdauer nur geringfügig schwächer ist als die Haftungsdauer, welche für die Zusammensetzung nach Beispiel 1 gemessen wurde.

### Beispiel 3:

Im Folgenden werden die Eigenschaften von Haftcremen für verschiedene Mengen an Sojaölzugabe aufgezeigt, wobei die Summe des Olivenöls und des hydrierten Sojaöls jeweils ca. 32,597 Gew.-% der Gesamtmenge der Haftcreme ausmacht, d.h. konstant gehalten wurde. Die restlichen Inhaltsstoffe sind in den Mengen vorhanden, wie sie für Beispielen 1 aufgelistet sind.

| **Beispiel** | **hydriertes Sojaöl* in Gewichtsprozent bezogen auf die Haftcreme** | | | **Haftungsdauer** | **Langzeitstabilität** |
|---|---|---|---|---|---|
| | **Total** | **vollhyd.** | **partiell hyd.** | | |
| 3.1 | 0.02 | 0.02 | - | | Mässig (<1 Monate) |
| 3.2 | 0.05 | 0.05 | - | | Mässig (<1 Monate) |
| 3.3 | 0.07 | 0.07 | - | | Mässig (<1 Monate) |
| 3.4 | 0.12 | 0.02 | 0.1 | | Mässig (<1 Monate) |
| 3.5 | 0.15 | 0.05 | 0.1 | | Mässig (<1 Monate) |
| 3.6 | 0.17 | 0.07 | 0.1 | | Mässig (<1 Monate) |
| 3.7 | 0.4 | 0.33 | 0.07 | >24 Stunden | Gut (≅2 Monate) |
| 3.8 | 0.4 | 0.33 | 0.07 | >24 Stunden | Gut (≅2 Monate) |
| 3.9 | 0.78 | 0.68 | 0.1 | >24 Stunden | Gut (≅2 Monate) |
| 3.10 | 1.54 | 1.37 | 0.17 | >24 Stunden | Sehr gut (>2 Monate) |
| 3.11 | 1.54 | 1.37 | 0.17 | >24 Stunden | Sehr gut (>2 Monate) |
| 3.12 | 2.255 | 2.05 | 0.205 | ca. 12 Stunden | Sehr gut (>2 Monate) |

| | | | | | |
|---|---|---|---|---|---|
| * Steigschmelztemperatur des vollhydrierten Sojaöls: 66-72°C, Steigschmelztemperatur des teilhydrierten Sojaöls: 36-42°C. | | | | | |

Der Langzeitstabilitätstest basiert auf einer visuellen Beurteilung der Phasenseparation. Die Haftfestigkeit wurde durch praktische Anwendung der Haftcreme beim Tragen von Zahnprothesen bestimmt.

Die Zusammensetzung einer Haftcreme für Zahnprothesen hat besonders vorteilhafte Langzeitstabilität bei einem Gewichtsverhältnis vom zweiten pflanzlichen Öl oder Fett zum ersten pflanzlichen Öl oder Fett im Bereich von 1:450 bis 1:10. Eine sehr gute Langzeitstabilität bei gleichzeitig einer Haftungsdauer von wenigstens 24 Stunden wurde im Bereich zwischen 0,17 Gew.-% und 2,255 Gew.-% Zugabe von hydriertem Sojaöl gefunden. Daraus ergibt sich, dass die Zusammensetzung einer Haftcreme für Zahnprothesen ganz besonders vorteilhaft ist, wenn das Gewichtsverhältnis vom zweiten pflanzlichen Öl oder Fett zum ersten pflanzlichen Öl oder Fett im Bereich von 1:190 bis 1:13, weiter bevorzugt von 1:150 bis 1:14, weiter bevorzugt von 1:100 bis 1:15, weiter bevorzugt von 1:50 bis 1:16, liegt.

## Patentansprüche

1. Cremebasis, insbesondere Haftcremebasis für Zahnprothesen, enthaltend wenigstens ein erstes pflanzliches Öl oder Fett, dessen Fettsäuregehalt zumindest zu 20 Gew.-% aus ungesättigten Fettsäuren besteht,
**dadurch gekennzeichnet,**
- **dass** der Fettsäuregehalt des ersten pflanzlichen Öls oder Fetts mehrheitlich aus Fettsäuren mit einer Kettenlänge von 12 oder mehr C-Atomen besteht und
- **dass** die Cremebasis wenigstens ein zweites pflanzliches Öl oder Fett enthält, welches hydriert ist, wobei das zweite pflanzliche Öl oder Fett ausgewählt ist aus der Gruppe bestehend aus hydriertem Sojaöl, hydriertem Rizinusöl oder einer Mischung davon.

2. Cremebasis nach dem vorangehenden Anspruch, **dadurch gekennzeichnet, dass** das erste pflanzliche Öl oder Fett zu 70-97 Gew.-%, bevorzugt zu 80-96 Gew.-%, weiter bevorzugt zu 85-95 Gew.-%, bezogen auf die Gesamtmenge der Cremebasis enthalten ist.

3. Cremebasis nach einem der vorangehenden Ansprüche, **dadurch gekennzeichnet, dass** das erste pflanzliche Öl oder Fett im Wesentlichen Olivenöl ist.

4. Cremebasis nach einem der vorangehenden Ansprüche, **dadurch gekennzeichnet, dass** das Gewichtsverhältnis vom zweiten pflanzlichen Öl oder Fett zum ersten pflanzlichen Öl oder Fett bei 1:450 oder mehr insbesondere im Bereich von 1:450 bis 1:10, bevorzugt bei 1:190 oder mehr, insbesondere im Bereich von 1:190 bis 1:13, weiter bevorzugt bei 1:150 oder mehr, insbesondere im Bereich von 1:150 bis 1:14, weiter bevorzugt bei 1:100 oder mehr, insbesondere im Bereich von 1:100 bis 1:15, weiter bevorzugt bei 1:50 oder mehr, insbesondere im Bereich von 1:50 bis 1:16, liegt.

5. Cremebasis nach einem der vorangehenden Ansprüche, **dadurch gekennzeichnet, dass** Trihydroxistearin darin enthalten ist, bevorzugt in einer Menge von 0.001 Gew.-% bis 5 Gew.-%, insbesondere in einer Menge von 0.01 Gew.-% bis 3 Gew.-%, bezogen auf die Gesamtmenge der Cremebasis darin enthalten ist.

6. Cremebasis nach einem der vorangehenden Ansprüche, **dadurch gekennzeichnet, dass** Siliziumdioxid darin enthalten ist, bevorzugt in einer Menge von 0.001 Gew.-% bis 5 Gew.-%, weiter bevorzugt in einer Menge von 0.1 Gew.-% bis 4 Gew.-%, weiter bevorzugt in einer Menge von 0.5 Gew.-% bis 3 Gew.-%, bezogen auf die Gesamtmenge der Cremebasis.

7. Cremebasis nach einem der vorangehenden Ansprüche, **dadurch gekennzeichnet, dass** Phosphoglyceride darin enthalten sind,
- wobei die Phosphoglyceride bevorzugt in einer Menge von 0.001 Gew.-% bis 3 Gew.-%,weiter bevorzugt von 0.001 Gew.-% bis 2 Gew.-% und weiter bevorzugt von 0.01 Gew.-% bis 1 Gew.-%, bezogen auf die Gesamtmenge der Cremebasis darin enthalten sind, und/oder
- wobei die Phosphoglyceride vorzugsweise aus der Gruppe der Lecithine ausgewählt sind, insbesondere bevorzugt der Soyalecithine.

8. Cremebasis nach einem der vorangehenden Ansprüche, **dadurch gekennzeichnet, dass** die Menge an Trihydroxystearin, Phosphoglyceride und Siliziumdioxid in Summe die Menge von maximal 10 Gew.-%, bevorzugt maximal 7 Gew.-%, weiter bevorzugt maximal 5 Gew.-%, bezogen auf die Gesamtmenge der Cremebasis nicht übersteigt.

9. Cremebasis nach einem der vorangehenden Ansprüche, **dadurch gekennzeichnet, dass** der Fettsäuregehalt des zweiten pflanzlichen Öls oder Fetts aus bis maximal 1 Gew.-% mehrfachungesättigten Fettsäuren besteht.

10. Cremebasis nach einem der vorangehenden Ansprüche, **dadurch gekennzeichnet, dass**
- die Steigschmelztemperatur des zweiten pflanzlichen Öls oder Fetts zumindest dem Mittelwert zwischen der Steigschmelztemperatur des zweiten pflanzlichen Öls oder Fetts in seinem noch unbehandelten, d.h. nichthydrierten, Zustand und der Steigschmelztemperatur in seinem vollhydrierten Zustand entspricht oder höher ist und/oder
- die Steigschmelztemperatur des zweiten pflanzlichen Öls oder Fetts zumindest im oberen Viertel des Temperaturbereichs zwischen der Steigschmelztemperatur des zweiten pflanzlichen Öls oder Fetts in seinem noch unbehandelten, d.h. nichthydrierten, Zustand und der Steigschmelztemperatur des zweiten pflanzlichen Öls oder Fetts in seinem vollhydrierten Zustand liegt.

11. Cremebasis nach einem der vorangehenden Ansprüche, **dadurch gekennzeichnet, dass** das zweite pflanzliches Öl oder Fett im Wesentlichen vollhydriert ist.

12. Cremebasis nach einem der vorangehenden Ansprüche, **dadurch gekennzeichnet, dass**
- der Fettsäuregehalt des ersten pflanzlichen Öls oder Fetts mehrheitlich, vorzugsweise zu wenigstens 65 Gew.-%, weiter bevorzugt zu wenigstens 80 Gew.-%, aus Fettsäuren mit einer Kettenlänge von 16-18 C-Atomen besteht,
- der Fettsäuregehalt des ersten pflanzlichen Öls oder Fetts zumindest zu 40 Gew.-%, weiter bevorzugt zumindest zu 50 Gew.-%, weiter bevorzugt zumindest zu 60 Gew.-%, weiter bevorzugt zumindest zu 70 Gew.-%, weiter bevorzugt zumindest zu 80 Gew.-%, aus ungesättigten (d.h. mehrfach- und einfachungesättigte) Fettsäuren besteht,
- der Fettsäuregehalt des ersten pflanzlichen Öls oder Fetts, zumindest zu 8 Gew.-%, bevorzugt zumindest zu 9 Gew.-%, weiter bevorzugt zumindest zu 10 Gew.-%, aus mehrfachungesättigten Fettsäuren besteht und/oder
- der Fettsäuregehalt des ersten pflanzlichen Öls oder Fetts, maximal zu 30 Gew.-%, bevorzugt maximal zu 25 Gew.-%, weiter bevorzugt maximal zu 20 Gew.-%, aus mehrfachungesättigten Fettsäuren besteht.

13. **Zusammensetzung** einer Haftcreme für Zahnprothesen, enthaltend
a) wenigstens ein wasserlösliches Polymer ausgewählt aus der Gruppe der Cellulosederivate, und
b) wenigstens ein Alkylvinylether/Maleinsäureanhydrid-Copolymer, **dadurch gekennzeichnet, dass** die Zusammensetzung weiter
c) die Cremebasis nach einem der Ansprüche 1-12 beinhaltet.

14. Zusammensetzung einer Haftcreme für Zahnprothesen nach dem vorangehenden Anspruch 12, **dadurch gekennzeichnet, dass** diese bezogen auf die Gesamtmenge der Haftcremezusammensetzung wenigstens zu 25 Gew.-% die Cremebasis, zu 10 bis 45 Gew.-% das wenigstens eine wasserlösliche Polymer ausgewählt aus der Gruppe der Cellulosederivate, und zu 25 bis 45 Gew.-% das wenigstens eine Alkylvinylether/Maleinsäureanhydrid-Copolymer enthält.

15. Cremebasis nach einem der vorangehenden Ansprüche 1-12, **dadurch gekennzeichnet, dass** es sich um eine Cremebasis für kosmetische, medizinische oder dentale Produkte handelt, vorzugsweise zur Anwendung auf Haut und im Mund.

16. Cremebasis nach einem der vorangehenden Ansprüche 1-12 oder 15, **dadurch gekennzeichnet, dass** das wenigstens eine hydrierte pflanzliche Öl oder Fett teilhydriertes pflanzliches Öl oder Fett und vollhydriertes pflanzliches Öl oder Fett beinhaltet.

## Claims

1. Cream base, in particular adhesive cream base for dental prostheses, containing at least a first vegetal oil or fat, the fatty acid content of which consists at least of 20% by weight of unsaturated fatty acids,
**characterized in**
- **that** the fatty acid content of the first vegetal oil or fat predominantly consists of fatty acids with a chain length of 12 or more carbon atoms and
- **that** the cream base contains at least a second vegetal oil or fat which is hydrogenated, wherein the second vegetal oil or fat is selected from the group consisting in hydrogenated soy oil, hydrogenated castor oil or in a mixture thereof.

2. Cream base according to the preceding claim, **characterized in that** the first vegetal oil or fat is contained up to 70-97% by weight, preferably up to 80-96% by weight, more preferably up to 85-95% by weight, based on the total amount of the cream base.

3. Cream base according to one of the preceding claims, **characterized in that** the first vegetal oil or fat is substantially olive oil.

4. Cream base according to one of the preceding claims, **characterized in that** the weight ratio of the second vegetal oil or fat to the first vegetal oil or fat is 1:450 or more, in particular in the range of 1:450 to 1:10, preferably 1:190 or more, in particular in the range of 1:190 to 1:13, more preferably 1:150 or more, in particular in the range of 1:150 to 1:14, more preferably 1:100 or more, in particular in the range of 1:100 to 1:15, more preferably 1:50 or more, in particular in the range of 1:50 to 1:16.

5. Cream base according to one of the preceding claims, **characterized in that** trihydroxistearin is contained therein, preferably in a quantity of 0.001% to 5% by weight, in particular in a quantity of 0.01% to 3% by weight, based on the total amount of the cream base.

6. Cream base according to one of the preceding claims, **characterized in that** silicon dioxide is contained therein, preferably in a quantity of 0.001% to 5% by weight, more preferably in a quantity of 0.1% to 4% by weight, still more preferably in particular in a quantity of 0.5% to 3% by weight, based on the total amount of the cream base.

7. Cream base according to one of the preceding claims, **characterized in that** phosphoglycerides are contained therein,
- wherein the phosphoglyceride are contained therein preferably in a quantity of 0.001% to 3% by weight, more preferably of 0.001% to 2% by weight and still more preferably of 0.01% to 1% by weight, based on the total amount of the cream base and/or
- wherein the phosphoglycerides are preferably selected from the group of the lecithins, in particular preferably of the soy lecithins.

8. Cream base according to one of the preceding claims, **characterized in that** the quantity of trihydroxystearin, phosphoglycerides and silicon dioxide in total does not exceed the quantity of not more than 10% by weight, preferably not more than 7% by weight, more preferably not more of 5% by weight, based on the total amount of the cream base.

9. Cream base according to one of the preceding claims, **characterized in that** the fatty acid content of the second vegetal oil or fat consists of not more than 1 % by weight of polyunsaturated fatty acids.

10. Cream base according to one of the preceding claims, **characterized in that**
- the slip melting temperature of the second vegetal oil or fat corresponds to or is higher than at least the mean value between the slip melting temperature of the second vegetal oil or fat in its still untreated, i.e. not hydrogenated, state and corresponds to or is higher than the slip melting temperature in its fully hydrogenated state and/or
- the slip melting temperature of the second vegetal oil or fat is situated at least in the upper quarter of the temperature range between the slip melting temperature of the second vegetal oil or fat in its still untreated, i.e. not hydrogenated, state and the slip melting temperature of the second vegetal oil or fat in its fully hydrogenated state.

11. Cream base according to one of the preceding claims, **characterized in that** the second vegetal oil or fall is substantially fully hydrogenated.

12. Cream base according to one of the preceding claims, **characterized in that**
- the fatty acid content of the first vegetal oil or fat predominantly consists of fatty acids with a chain length of 16-18 carbon atoms, preferably at least of 65% by weight, more preferably of at least 80% by weight,
- the fatty acid content of the first vegetal oil or fat consists of unsaturated (i.e. polyunsaturated and monounsaturated) fatty acids at least 40% by weight, more preferably of at least 50% by weight, still more preferably of at least 60% by weight, still more preferably of at least 70% by weight, still more preferably of 80% by weight,
- the fatty acid content of the first vegetal oil or fat consists of polyunsaturated fatty acids of at least 8% by weight, more preferably of at least 9% by weight, still more preferably of at least 10% by weight and/or
- the fatty acid content of the first vegetal oil or fat consists of polyunsaturated fatty acids of not more than 30% by weight, more preferably of not more than 25% by weight, still more preferably of not more than 20% by weight.

13. Composition of an adhesive cream for dental prostheses containing
a) at least one water soluble polymer selected from the group of the cellulose derivatives and
b) at least one alkylvinylether/maleic anhydride copolymer,
**characterized in that** the composition
c) further contains the cream base according to one of the claims 1-12.

14. Composition of an adhesive cream for dental prostheses according to the preceding claim 12, characterized that it contains, based on the total amount of the adhesive cream composition, at least 25% by weight of the cream base, 10 to 45% by weight of the at least one water soluble polymer selected from the group of the cellulose derivatives and at least one alkylvinylether/maleic anhydride copolymer.

15. Cream base according to one of the preceding claims 1 to 12, **characterized in that** it is a cream base for cosmetic, medical or dental products, preferably for applying onto the skin and in the mouth.

16. Cream base according to one of the preceding claims 1 to 12 or 15, **characterized in that** the at least one hydrogenated vegetal oil or fat contains partially hydrogenated vegetal oil or fat and fully hydrogenated vegetal oil or fat.

## Revendications

1. Base de crème, en particulier base de crème adhésive pour prothèses dentaires, contenant au moins une première huile ou graisse végétale dont la teneur en acides gras est d'au moins 20% en poids en acides gras insaturés, **caractérisée en ce**
- **que** la teneur en acide gras de la première huile ou graisse végétale se compose majoritairement d'acides gras avec une longueur de chaîne de 12 atomes de carbone ou plus et
- **que** la base de crème contient au moins une seconde huile ou graisse végétale qui est hydrogénée, cependant que la seconde huile ou graisse végétale est sélectionnée dans le groupe comprenant l'huile de soja hydrogénée, l'huile de ricin hydrogénée ou un mélange de celles-ci.

2. Base de crème selon la revendication précédente, **caractérisée en ce que** la première huile ou graisse végétale est contenue pour 70-97% en poids, de préférence pour 80-96% en poids, de manière plus préférée pour 85-95% en poids par rapport à la quantité totale de base de crème.

3. Base de crème selon l'une des revendications précédentes, **caractérisée en ce que** la première huile ou graisse végétale est essentiellement de l'huile d'olive.

4. Base de crème selon l'une des revendications précédentes, **caractérisée en ce que** le rapport de poids de la seconde huile ou graisse végétale par rapport à la première huile ou graisse végétale est de 1:450 ou plus en particulier de l'ordre de 1:450 à 1:10, de préférence de 1:190 ou plus, en particulier de l'ordre de 1:190 à 1:13, de manière plus préférée de 1:150 ou plus, en particulier de l'ordre de 1:150 à 1:14, de manière encore plus préférée de 1:100 ou plus, en particulier de l'ordre de 1:100 à 1:15, de manière encore plus préférée de 1:50 ou plus, en particulier de l'ordre de 1:50 à 1:16.

5. Base de crème selon l'une des revendications précédentes, **caractérisée en ce que** de la trihydroxy-stéarine y est contenue, de préférence en une quantité de 0,001 à 5% en poids, en particulier en une quantité de 0,01 à 3% en poids, par rapport à la quantité totale de base de crème.

6. Base de crème selon l'une des revendications précédentes, **caractérisée en ce que** du dioxyde de silicium y est contenu, de préférence en une quantité de 0,001 à 5% en poids, en particulier en une quantité de 0,1 à 4% en poids, de manière plus préférée en une quantité de 0,5 à 3% en poids, par rapport à la quantité totale de base de crème.

7. Base de crème selon l'une des revendications précédentes, **caractérisée en ce que** des phosphoglycérides y sont contenus,
- cependant que les phosphoglycérides y sont contenus de préférence en une quantité de 0,001 à 3% en poids, de manière plus préférée de 0,001 à 2% en poids et de manière encore plus préférée de 0,01 à 1% en poids, par rapport à la quantité totale de base de crème et/ou
- cependant que les phosphoglycérides sont sélectionnés de préférence dans le groupe des lécithines, en particulier de préférence des sojalécithines.

8. Base de crème selon l'une des revendications précédentes, **caractérisée en ce que** la quantité de trihydroxy-stéarine, de phosphoglycérides et de dioxyde de silicium au total ne dépasse pas la quantité maximale de 10% en poids, d'au maximum de préférence 7% en poids, de manière plus préférée d'au maximum de 5% en poids maximum, par rapport à la quantité totale de base de crème.

9. Base de crème selon l'une des revendications précédentes, **caractérisée en ce que** la teneur en acides gras de la seconde huile ou graisse végétale est constituée d'au maximum 1% en poids d'acides gras polyinsaturés.

10. Base de crème selon l'une des revendications précédentes, **caractérisée en ce que**
- la température de montée en fusion de la seconde huile ou graisse végétale correspond au moins ou est supérieure à la valeur moyenne entre la température de montée en fusion de la seconde huile ou graisse végétale dans son état encore non traité, c'est-à-dire non hydrogéné, et la température de montée en fusion dans son état entièrement hydrogéné et/ou
- que la température de montée en fusion de la seconde huile ou graisse végétale se situe au moins dans le quart supérieur de la plage de température entre la température de montée en fusion de la seconde huile ou graisse végétale dans son état encore non traité, c'est-à-dire non hydrogéné, et la température de montée en fusion dans son état entièrement hydrogéné.

11. Base de crème selon l'une des revendications précédentes, **caractérisée en ce que** la seconde huile ou graisse végétale est substantiellement entièrement hydrogénée.

12. Base de crème selon l'une des revendications précédentes, caractérisée en ce **caractérisée en ce**
- **que** la teneur en acide gras de la première huile ou graisse végétale se compose majoritairement de préférence d'au moins 65% en poids, de manière plus préférée d'au moins 80% en poids d'acides gras, avec une longueur de chaîne de 16 à 18 atomes de carbone,
- la teneur en acide gras de la première huile ou graisse végétale se compose d'au moins 40% en poids d'acides gras insaturés (c'est-à-dire polyinsaturés ou monoinsaturés), de manière plus préférée d'au moins 50% en poids, de manière plus préférée d'au moins 60% en poids, de manière plus préférée d'au moins 70% en poids, de manière encore plus préférée d'au moins 80% en poids,
- la teneur en acides gras de la première huile ou graisse végétale se compose d'au moins 8% en poids d'acides gras polyinsaturés, de préférence d'au moins 9% en poids, de manière plus préférée d'au moins 10% en poids et/ou
- la teneur en acide gras de la première huile ou graisse végétale se compose au maximum de 30% en poids d'acides gras polyinsaturés, de préférence au maximum de 25% en poids, de manière plus préférée au maximum de 20% en poids.

13. Composition d'une crème adhésive pour prothèses dentaires contenant
a) au moins un polymère soluble dans l'eau sélectionné dans le groupe des dérivés de cellulose et
b) au moins un copolymère d'alkylvinyléther/d'anhydride maléique,
**caractérisée en ce que** la composition contient de plus
c) la base de crème selon l'une des revendications 1 à 12.

14. Composition d'une crème adhésive pour prothèses dentaires selon la revendication précédente 12, **caractérisée en ce que** celle-ci contient, par rapport à la quantité totale de la composition de crème adhésive, au moins 25% en poids de base de crème, de 10 à 45% en poids du au moins un polymère soluble dans l'eau sélectionné dans le groupe des dérivés de cellules et de 24 à 14% en poids du au moins un copolymère d'alkylvinyléther/d'anhydride maléique.

15. Composition d'une crème adhésive pour prothèses dentaires selon l'une des revendications précédentes 1 à 12, **caractérisée en ce que** qu'il s'agit d'une base de crème pour produits cosmétiques, médicaux ou dentaires, de préférence pour l'application sur la peau et dans la bouche.

16. Composition d'une crème adhésive pour prothèses dentaires selon l'une des revendications précédentes 1 à 12 ou 15 **caractérisée en ce que** la au moins une huile ou graisse végétale hydrogénée contient de l'huile ou de la graisse végétale partiellement hydrogénée et de l'huile ou de la graisse végétale entièrement hydrogénée.
